# EUROPEAN PATENT SPECIFICATION

(11) **EP 0 340 740 B1**
(45) Date of publication and mention of the grant of the patent: **15.11.1995**
(21) Application number: 89107958.4
(22) Date of filing: 02.05.1989
(51) Int. Cl.: C08G 65/00, C08L 71/00

(54) **Perfluoropolyethers containing a halogen different from fluorine and having an acid end group**
Perfluoropolyether, die ein anderes Halogen ausser Fluor enthalten und die eine Säureendgruppe haben
Perfluoropolyéthers contenant un halogène autre que le fluor et ayant des groupements terminaux acides

(30) Priority: 02.05.1988 IT 2040688
(43) Date of publication of application: 08.11.1989
(73) Proprietor: AUSIMONT S.p.A., I-20100 Milano (IT)
(72) Inventor: Marchionni, Giuseppe, Dr., I-20133 Milan (IT); Staccione, Anna, Dr., I-20144 Milan (IT)
(74) Representative: Barz, Peter, Dr.

(56) References cited:
- EP-A- 0 192 493
- EP-A- 0 195 946
- DE-A- 3 739 447
- US-A- 3 715 378

## Description

The present invention relates to perfluoropolyethers characterized in that they have, at one end, a functional acid group, such as acyl halide or a carboxyl group or derivatives thereof (salts, esters), and, at the other end, a perfluoroalkyl group containing at least one halogen atom different from fluorine.

The products of the invention are prepared by a photooxidation process, starting from perfluoropropene and/or tetrafluoroethylene and a minor amount of a thoroughly fluorohalogenated ethylene containing at least one halogen atom different from fluorine. The photooxidation product is then subjected to a thermal or photochemical treatment to eliminate the peroxide groups contained therein.

Processes for the photooxidation of perfluoroolefins, in particular C₃F₆ and/or C₂F₄, are known, see, e.g., GB-A-1,189,337, 1,104,482; US-A-3,683,027 and 3,715,378 of the applicant. By the known photooxidation of C₃F₆ and by subsequent thermal decomposition of the peroxide groups it is possible to obtain mixtures of products having a perfluoropolyether structure with neutral (perfluoroalkyl) and acid (acyl fluoride group, 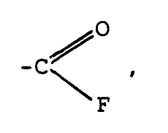
and derivatives) end groups, the latter being present in minor amounts (generally in amounts of not more than 30% of the perfluoroether products).

This is also due to the fact that during the photooxidation many acid end groups of the type 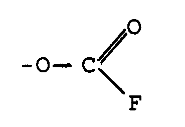
(fluoroformates) are formed, which, during the subsequent thermal treatment for the decomposition of the peroxide groups, result in ketone end groups (-CF₂COCF₃), having no acid function.

If the products to be obtained are the ones which have an acid end group, the separation thereof, in the form of a fraction having a high concentration of monoacid product, is very difficult and practically impossible, in particular in the case of products with a relatively high molecular weight.

At any rate, such processes are very expensive and of little interest from an industrial point of view.

It has now surprisingly been found that by use of a fully halogenated ethylene of the type specified below it is possible to prepare perfluoropolyethers with acid end groups in very high yields, i.e. equal to or higher than 90% by weight.

In the photooxidation process according to the present invention, the halogenated ethylene, which in the following will be represented by the formula X-CF = CF₂ (X = Cl, Br or I) reacts selectively, to afford the species: Rf-O-CF₂-CFXO°, which is quantitatively converted into Rf-O-CF₂-COF + X°. The species X° reacts further with olefin (b) CF₂ = CF-Y (Y = F, CF₃) resulting in the species XCF₂CFY, the latter starting a new chain.

Thus, the fluorohalogenated ethylene XCF = CF₂ acts as chain transfer agent (thereby allowing a molecular weight regulation) which generates two stable end groups, one containing Br, Cl or I, the other being acid.

The products of the present invention are characterized by having structures represented by the following general formulae.

When the starting perfluoroolefin is C₃F₆ the products obtained by means of the process of the present invention have the following general formula: 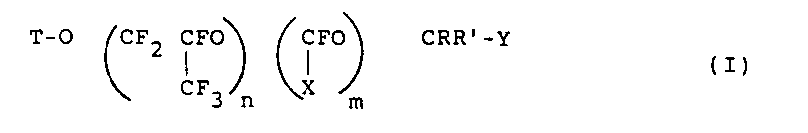
wherein X is F or CF₃, R and R', the same or different from each other, are selected from F, Cl, Br and I, preferably from F and Cl, Y is 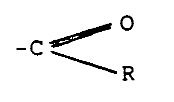
R being defined as above and preferably representing Cl or F, or Y is the corresponding carboxylic group -COOH or a salt (preferably of ammonium or alkali or alkaline earth metals, such as Na, K, Ca and Mg) or ester thereof (such as esters of aliphatic alcohols containing from 1 to 10, preferably 1 to 4, carbon atoms); T is a perfluoroalkyl group (preferably having 1 to 3 carbon atoms) wherein one or two F atoms are replaced by Cl and/or Br and/or I (preferably by Cl). Particularly, T may have the formulae ACF₂-, 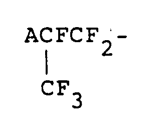
and ACF₂CF(CF₃)-, wherein A is Cl or Br or I (preferably Cl). n and m represent the average number of the corresponding units present in the chains.
In formula (I) n ranges from 1 to 15 with m/n being from 0.01 to 0.5,e.g. from 0.05 to 0.3,and the units 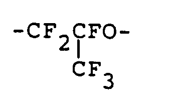
and 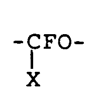
are statistically distributed along the chain. When C₂F₄ is used as starting perfluoroolefin, products having the following general formula can be obtained:

T'-O(CF₂CF₂O)ₚ (CF₂O)ₛ CRR' Y (II)

wherein: T' = ACF₂CF₂-, ACF₂-, ACF₂CF(A)-, R', R, A and Y being defined as for formula (I): p ranges from 1 to 20 and s/p being in the range of from 0.5 to 2.

If a mixture of C₂F₄ and C₃F₆ is employed, the resulting products have a structure of the following formula: 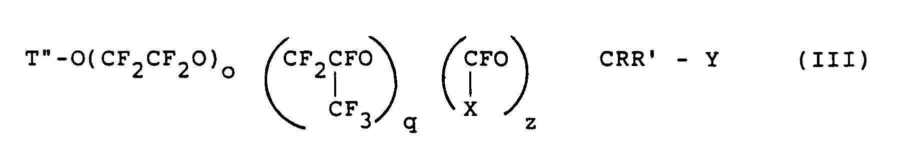
wherein: Y, X, R and R′ are the same as defined above for formula (I),
T˝ is equal to T or T′, o and q are numbers, including zero, such that $\text{o+q = 1 to 20}$ , $\text{z/o+q = 0.01 to 0.05}$ 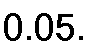 .

In formulae (II) and (III) above the units in brackets are also distributed at random within the chain and o,p,q,s and z are average values.

For n >15 in formula I, for p >20 in formula II and for o+p >20 in formula III, it is possible to obtain, in addition to the products of formulae I, II or III, also products corresponding to said formulae, wherein the end groups, however, are both perfluorohalogenalkyl groups or are both acyl halide (i.e. acid Y) groups. In such mixtures, which comprise at least 50% by weight of a product of formula I or II or III, the end group ratio T/Y is always about 1, i.e. they exhibit a functionality (in terms of acid groups Y) of about 1.

These mixtures can be used in the same fields as the products of the above formulae I, II and III.

The process for preparing the compounds according to the invention comprises the photooxidation of the perfluoroolefin (C₃F₆ and/or C₂F₄) with gaseous O₂ in the presence of a minor amount (preferably up to 50% by moles and, even more preferred, up to 20%) of a thoroughly (per)halogenated ethylene containing at least one atom selected from Cl, Br and I, by irradiation, with ultraviolet light (wavelength preferably ranging from 200 to 600 nm), of the liquid reaction mixture, maintained at a temperature of from -20°C to -100°C, preferably from -50°C to -60°C, in the presence or absence of solvents. The liquid reaction mixture, which initially consists of C₃F₆ and/or a chlorofluorocarbon solvent and/or other inert solvents, is maintained at the above temperature. The perhalogenated ethylene is introduced into the reaction mixture simultaneously with the O₂ flow. The fully halogenated ethylenes preferably contain 1 or 2 halogen atoms different from fluorine. Particularly preferred perhalogenated ethylenes for use in the process of the present invention are CF₂ = CFCl, CFCl = CFCl, CF₂ = CCl₂, CFCl = CCl₂, CF₂ = CFBr.

The photooxidation product may be subjected to a thermal treatment at a temperature of from 180° to 220°C, or to a photochemical treatment by irradiation with ultraviolet light for a period of time sufficient to decompose the peroxide groups which are present in the perfluoropolyether products.

Instead of carrying out the thermal or photochemical treatment directly with the photooxidation product, said treatment can be effected after hydrolysis of the group 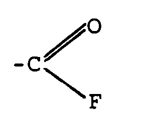
(which is converted to -COOH), thus resulting in a less volatile product.

By means of the process of the present invention it is possible to obtain, in particular starting from C₃F₆ a perfluoropolyether of formula (I) having a relatively low average molecular weight, suitable for most of the practical uses.

As regards possible applications, said perfluoropolyether in the first place may be used as fluorinated surfactant in the form of a salified monocarboxylic acid (ammonium salt or alkaline metal salt), or may be employed in the protection of monuments and of stony materials in general against pollutants and atmospheric agents, as described in EP-A-215492.

If C₂F₄ is utilized as starting perfluoroolefin, it is possible to obtain photooxidation products having a relatively low or in any case controlled average molecular weight, which is much lower than the molecular weight obtainable in the absence of perhalogenated ethylene, and is suitable for most of the possible applications. Therefore specific treatments (scission) for reducing the high molecular weight can be avoided.

The available methods of analysis, for example N.M.R., mass spectrophotometry and determination of the halogen (other than fluorine) content do not reveal that oxyalkylene units derived from the perhalogenated ethylene used in the photooxidation are present in the chain. Only in the synthesis of perfluoropolyether products having a high molecular weight, for example higher than 2000, it is assumed that negligible amounts, generally below 3% of the total, of the oxyalkylene units containing one or more halogen atoms different from fluorine may be situated along the chain: however, the analysis methods indicated above do not allow an exact determination of small amounts of said units in the chain.

The following examples merely illustrate the present invention and are not to be construed as a limitation of the scope thereof.

### EXAMPLES 1-4 - Photooxidation of C₃F₆ + CFCl = CF₂

Into a cylindrical glass reactor (volume = 500 ml, optical path = 1 cm), equipped with an inner coaxial quartz sheath and furthermore equipped with a dipping pipe for the introduction of gases, a sheath with a thermocouple for measuring the internal temperature and a reflux condenser maintained a a temperature of -80°C, 800 g of C₃F₆ were introduced at a temperaturer of -60°C. A gas mixture of 27 (26) l/h of O₂ and 3 (4) l/h of C₂F₃Cl was bubbled into the reactor through the dipping pipe. By means of a cooling bath placed around the reactor the temperature of the liquid reaction phase was maintained, for the whole duration of the experiment, at a predetermined value which is indicated in Table 1 for each example.

After having introduced into the quartz sheath an ultravioletray lamp HANAU® TQ 150 (47 W, wavelength ranging from 200 to 300 nm), the lamp was switched on and the irradiation and the introduction of the reactive gases were continued for 5 hours.

After a 5-hour irradiation, the lamp was switched off, the mixture was degassed and the unreacted C₃F₆ was recovered from the reactor by evaporation at room temperature. Thus an oily polymeric residue was obtained. Said residue was subjected to iodometric analysis in order to determine the active (peroxide) oxygen content, and to ¹⁹F-NMR analysis, which revealed that the residue was composed of polyether chains of the type: 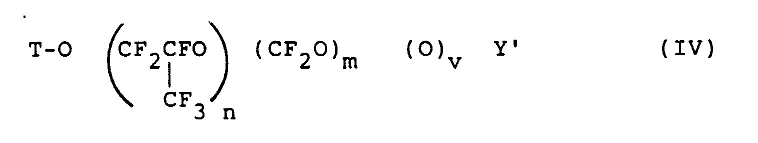
wherein: T = 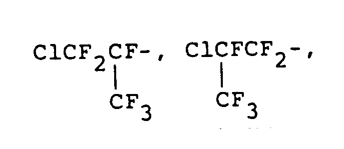
ClCF₂- with a pronounced prevalence of the first two types of end groups;
Y' = 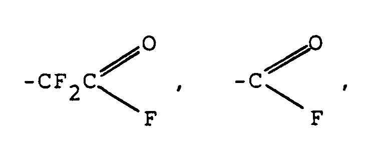
the latter type being present in an amount of <5% of the total of the end groups Y'. The m/n ratio was very low ( <0.05). The viscosity of the product at 20°C, indicated in Table 1 for each example, was determined by means of an Ostwald-Fenske viscosimeter. The infrared sepctra showed bands at 1884 cm⁻¹ which are typical for groups of the formula 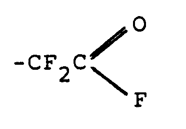
Reaction conditions and characteristics of the resulting oily products are reported in Table 1.

**TABLE 1**

| Example No. | T(°C) | Time (h) | O₂ (l/h) | CTFE (l/h) | Amount of Product (g/h) | P.O (*) | Visc. (cSt) |
|---|---|---|---|---|---|---|---|
| 1 | -60 | 5 | 27 | 3 | 66 | 0.6 | 6.9 |
| 2 | -60 | 5 | 27 | 3 | 72 | 0.5 | 5.7 |
| 3 | -60 | 5 | 26 | 4 | 74 | 0.8 | 7.3 |
| 4 | -50 | 5 | 27 | 3 | 65 | 0.6 | 4.5 |

| | | | | | | | |
|---|---|---|---|---|---|---|---|
| (*) peroxide oxygen in % by weight. | | | | | | | |

The mass spectrometric analysis confirmed both the presence of the end groups revealed by the NMR analysis, and the absence of units -CFCl-CF₂O- in the chain. According to the NMR analysis, the product of example 2 exhibited a m/n ratio of 0.01 and a molecular weight of 960.

The chlorine analysis indicated a Cl content of 4.2% by weight. Assuming that the product contained only one chlorine atom per chain, as reflected by the above formula, a molecular weight equal to 840 was calculated.

### Example 5 - Hydrolysis and thermal treatment

The product obtained in example 2 was treated with a H₂O-saturated nitrogen flow in order to hydrolize the predominant end groups of formula 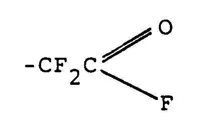
( > 95% with respect to the total of end groups Y′) to -CF₂COOH and the end groups 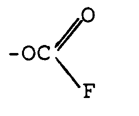
(present in an amount of <5%) to

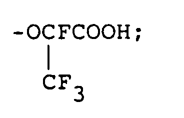

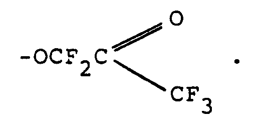

Thereafter, the hydrolized product was thermally treated by heating it up to 210°C in order to eliminate the peroxide groups. The thermal treatment was effected after the hydrolysis in order to avoid a high temperature treatment of a product having too high a volatility. 313.5 g of a non-peroxy product were obtained (yield = 95%).

### Example 6 - Distillation of the product

An amount of 71 g of the product obtained in example 5 was subjected to distillation at atmospheric pressure in order to obtain information regarding the molecular weight distribution. The results are reported in Table 2 which shows that 50% of the distillate had an average molecular weight ranging from 400 to 700.

**TABLE 2**

| Boiler temperature T (°C) | B.P. of distillate T (°C) | Amount of Fraction (g) | M.W. |
|---|---|---|---|
| 113 | 80 | - | - |
| 190 | 115 | 7.2 | 180 |
| 245 | 170 | 7.1^{*} | 400^{*} |
| 262 | 190 | 7.3 | 540 |
| 290 | 194 | 7.0 | 540 |
| 320 | 209 | 7.4 | 540 |
| 360 | 218 | 7.2 | 680 |
| Res. | - | 27.1 | 700 |

| | | | |
|---|---|---|---|
| * The fraction distilled at 170°C, the M.W. of which, determined by gas chromatography, was equal to 400, contained 8.1% of chlorine. Assuming that the product contained only one atom of chlorine per molecule, a molecular weight equal to 440 was calculated. Acidimetric titration indicated an equivalent weight of 410, while based on the NMR analysis a M.W. of 400 could be calculated. | | | |

### EXAMPLE 7 - Evaluation of the neutral products

a) To a first portion of 184.2 g of the product obtained according to example 5 120 ml of water and 15 g of CaO were added and the resulting mixture was kept under stirring for 6 hours. The mixture thus obtained was then subjected to distillation at atmospheric pressure and a head temperature of 90 to 95°C.
   From the distillate, 7.4 g of perfluoropolyether oil were demixed, for which, on the basis of the ¹⁹F-NMR spectrum, a molecular weight of 1100 was calculated. Infrared analysis confirmed that said oil did not contain carbonyl groups. A Cl amount equal to 7.7% by weight was determined, corresponding to an average molecular weight of 920, assuming that two chlorine atoms per molecule were present.
b) A portion of 21.2 g of product as such, obtained in example 2, was dissolved in 100 ml of methanol and percolated in a column (⌀ = 100 mm; h = 1000 mm) filled with 100 ml (about 50 g) of a strongly basic ion exchange resin (Amberlite® IRA 400) which had previously been activated with NaOH and washed with H₂O.
   The resin fixed the acids and, after concentration of the eluate, 0.78 g (3.7% by weight of the starting product) of neutral product were collected.

### EXAMPLE 8 - Purification and characterization of the acids

The salts resulting from the procedure of example 7 a) were treated with concentrated HCl and thereafter were heated to a temperature of 70°C; keeping them at this temperatures for 5 hours under stirring. Upon completion of the reaction, 161 g (equal to 87.3%) of acid perfluoropolyethers were separated, which, subjected to structural analysis, showed the following characteristics:
The molecular weight, determined by NMR analysis, was 860, with a molecular structure in accordance with the above general formula (examples 1-4), wherein 95% of the groups Y′ equal -CF₂COOH.
The chlorine content was 3.7% by weight, corresponding to a molecular weight of 960, assuming that the product contained one Cl atom per molecule. Acidimetric titration indicated an equivalent weight of 950.

### EXAMPLES 9-19 - Photooxidation of C₂F₄ + CFCl = CF₂

Into a cylindrical glass reactor (diameter = 80 mm, volume = about 500 ml), equipped with a coaxial inner quartz sheath (diameter 20 mm) and furthermore equipped with a dipping pipe for the introduction of gases and a reflux condenser maintained at a temperature of -80°C, 500 ml of A-12 (CF₂Cl₂) were introduced. Through the dipping pipe, a gaseous mixture of oxygen, C₂F₄ and ClC₂F₃ was bubbled into the reactor. By means of a cooling bath placed around the reactor, the temperature of the reacting liquid phase was maintained at the temperature indicated in Table 3 for the whole duration of the test. After having introduced into the quartz sheath an ultraviolet-ray lamp type HANAU® TQ 150 (47 W, wavelength ranging from 200 to 300 nm), the lamp was switched on and irradiation and feeding of the reagents were continued for 5 hours. The gases leaving the reactor were eliminated after having undergone an alkaline washing. After a five-hour irradiation, the lamp was switched off and the solvent was removed from the reactor by evaporation at room temperature. Thus, an oily polymeric residue was obtained. Said residue was subjected to iodometric analysis, in order to determine the active oxygen content, and to ¹⁹F-NMR analysis, which revealed that the residue was composed of polyether chains of the type:

T'-O(CF₂CF₂O)ₚ(CF₂O)ₛ(O)ᵥY' (V)

with
T' = ClCF₂CF₂ -; ClCF₂ -
Y' = CF₂COF; -COF
The s/p ratio depended on the reaction conditions (temperature) and ranged from 0.5 to 2.
The product viscosity at 20°C was determined by means of an OSTWALD-FENSKE viscosimeter. Reaction conditions and characteristics of the oils so produced are indicated in Table 3.

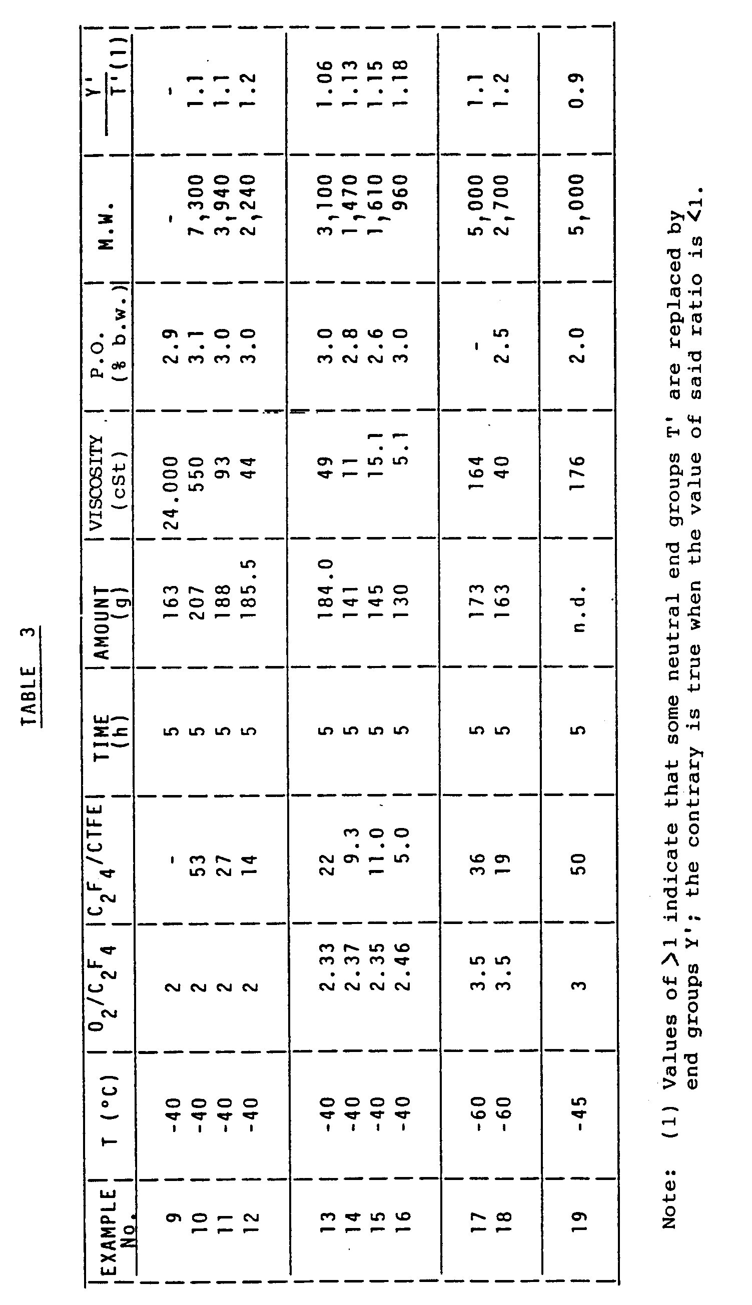

### EXAMPLE 20 - Hydrolysis, thermal treatment and esterification

The product obtained in example 19 was thermally treated (up to a temperature of 220°C, for 3 hours, with a weight loss of 20%) in order to eliminate peroxide oxygen. After this treatment, the viscosity of the sample was 56 cSt. A portion of the resulting product was treated with humid air until the hydrolysis of the end groups (consisting of 100% of -CF₂COF) was complete. After this treatment the product, according to ¹⁹F-NMR analysis, was composed of perfluoropolyether chains of the above formula V, wherein v = o, Y′ = -CF₂COOH and T′ = -CF₂Cl and -CF₂CF₂Cl, and wherein the Y′/T′ ratio was 0.9 and the s/p ratio equaled 1.4, the average molecular weight being about 5000.
A thermally treated portion of the product was esterified with methanol. The resulting esterified product, in which Y′ = COOCH₃. was subjected to ¹⁸F-and ¹H-NMR analyses which confirmed the data reported above.

### EXAMPLES 21-23 - Photooxidation of C₃F₆ + C₂F₄ - CFCl = CF₂

In a photochemical reactor as described in example 1 and containing the same amount of C₃F₆ a photosynthesis was carried out after having bubbled into the reactor, through the dipping pipe, a gaseous mixture of C₂F₄ and C₂ClF₃.
At the end of the experiment the lamp was switched off, the reactor was degassed and the unreacted C₃F₆ was recovered from the reaction mixture by evaporation at room temperature.
Thus an oily polymeric residue was obtained. This residue was subjected to iodometric analysis, in order to determine the active oxygen content, and to ¹⁹F-NMR analysis, which revealed that it was composed of polyether chains of the type: 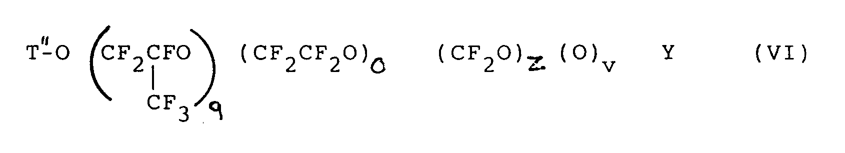
wherein: T'' = 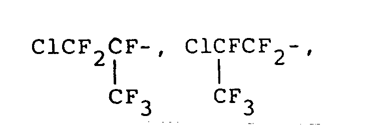
ClCF₂-, ClCF₂CF₂-
with a pronounced prevalence of the first two types of end groups;
Y =-CF₂COF, -COF
with a pronounced predominance of the first type of end groups. In these examples, the o/q ratio ranged from 0.5 to 3 and the $\text{z/o+q}$ ratio was from 0.01 to 0.05.
Table 4 shows the reaction conditions as well as the characteristics of the oils produced.

**TABLE 4**

| Example No. | T(°C) | Time (h) | O₂ (l/h) | CTFE (l/h) | TFE (l/h) | Amount (g/h) | P.O. (%b.w.) | Visc. (cSt) |
|---|---|---|---|---|---|---|---|---|
| 21 | -40 | 5 | 27 | 1 | 2 | 84 | 0.78 | 28 |
| 22 | -60 | 5 | 27 | 1 | 2 | 54 | 0.62 | 31 |
| 23 | -60 | 3 | 27 | 1 | 2 | 51 | 0.77 | 19 |

### EXAMPLE 24 - Photooxidation of C₃F₆ + CFCl = CFCl

A photosynthesis at a temperature of -60°C was carried out in a photochemical reactor like the one described in example 1, into which, through the dipping pipe, a gaseous mixture (27 l/h) was bubbled, in which the O₂/C₂F₂Cl₂ ratio (by volume) was equal to 3.
After a two-hour reaction, the lamp was switched off, the reactor was degassed and the unreacted C₃F₆ was recovered from the reactor by evaporation at room temperature. Discharged was a product (51 g) which, according to iodometric analysis, exhibited an active oxygen content of 0.34 % b.w. The ¹⁹F-NMR analysis revealed a composition of polyether chains of the type: 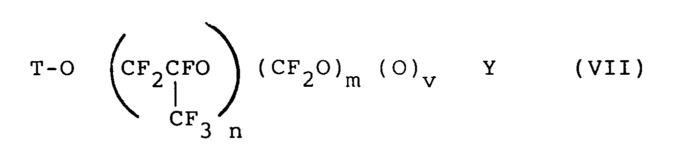
Y = -OCFClCOF, -OCOF
with a pronounced prevalence of the first type;
T = 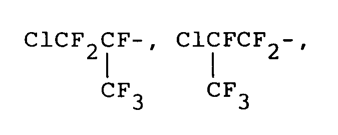
ClCF₂-, ClCF₂CFCl-
with a pronounced prevalence of the first two types of end groups.
The m/n ratio was of the order of 0.015. The product viscosity, determined at 20°C by means of an OSTWALD-FENSKE viscosimeter, was 3.3 cSt.

### EXAMPLE 25 - Photooxidation of C₃F₆ with CFBr = CF₂

In a photochemical reactor like the one described in example 1, charged with 800 g of C₃F₆, a photosynthesis was carried out at a temperature of -64°C. Oxygen and BrC₂F₃,in a ratio of 2.4/l by volume, were separately bubbled into the reaction liquid at a total flow-rate of 2 l/h. The gaseous reagents had previously been diluted with helium (18 l/h). After 5 hours, the lamp was switched off and the unreacted C₃F₆ was recovered by evaporation at room temperature. 43.1 g of an oily product were obtained. The iodometric analysis revealed an active oxygen content of 0.43%.
The product had a viscosity of 6.95 cSt, and the ¹⁹F-NMR analysis indicated that the polyether was composed of perfluoropolyether structures of the type: 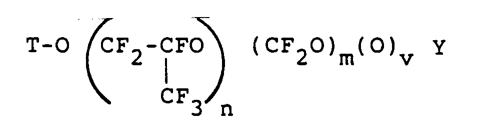
wherein: T = 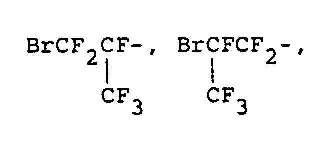
CF₃-
with a pronounced prevalence of the first two types;
Y = -CF₂COF, -COF
with pronounced prevalence of the first type of end groups, having an average molecular weight of 800 and a m/n ratio of 0.05.

## Claims (Claims for the following Contracting State(s): BE, CH, DE, FR, GB, LI, NL, SE)

1. Perfluoropolyethers comprising sequences of statistically distributed perfluorooxyalkylene units selected from 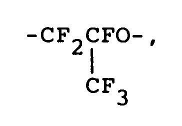 -CF₂CF₂O-, -CF₂O- and 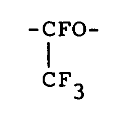 and having an acid group or derivative thereof, the other end group being a perhaloalkyl group containing one or two halogen atoms different from fluorine, said perfluoropolyethers being represented by the following general formulae: 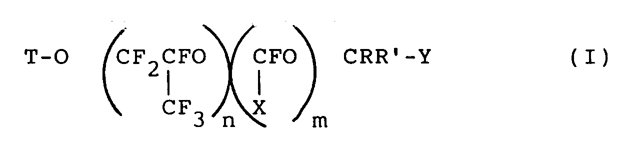 wherein: X is F or CF₃; R and R', the same or different from each other, are selected from F, Cl, Br and I;
Y is 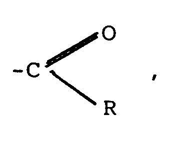 R being the same as defined above, or Y is the corresponding carboxyl group -COOH or a salt or ester group derived therefrom;
T is a perhaloalkyl group containing one or two atoms selected from Cl, Br and I and preferably represents ACF₂-, 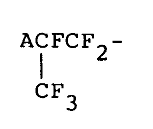 and ACF₂CF(CF₃)-, wherein A is Cl, Br or I;
n ranges from 1 to 15;
and m/n is in the range of from 0.01 to 0.5;
T'-O (CF₂CF₂O)ₚ (CF₂O)ₛ CRR' Y (II)
wherein: T' = ACF₂CF₂-, ACF₂-, ACF₂CF(A)-, R, R', A and Y are the same as defined above for formula (I); p ranges from 1 to 20 and s/p is from 0.5 to 2; 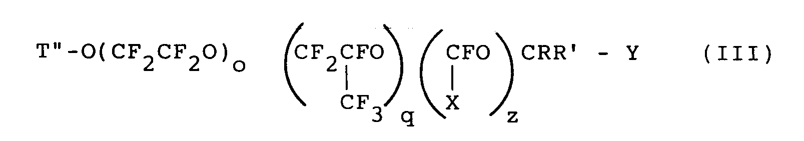 wherein: Y, X, R and R' are the same as defined above for formula (I);
T'' is equal to T or T', o and q are numbers, zero included, such that $\text{o+q = 1 to 20}$ , $\text{z/o+q}$ ranges from 0.01 to 0.05 n, m, p, s, o, q and z are average numbers.

2. Process for preparing the perfluoropolyethers of claim 1, comprising the photooxidation of C₃F₆ and/or C₂F₄ with gaseous oxygen, in the presence of a perhalogenated ethylene containing at least one halogen atom different from fluorine, at a temperature from -20° to -100°C, by irradiation of the liquid reaction mixture with ultraviolet light and the subsequent elimination of the peroxide groups by heat-treatment at 180° to 220°C, or the subsequent photochemical treatment with ultraviolet radiation, and optionally also comprising the hydrolysis, salification or alcoholysis of the acyl halide group formed.

3. Perfluoropolyether mixtures, comprising at least 50% by weight of perfluoropolyethers of formulae I, II or III of claim 1, besides perfluoropolyethers corresponding to the above formulae, except that both of the end groups are either equal to T or T' or are equal to Y, said mixtures being characterized by a ratio of the number of end groups of types T, T and T'' to the number of end groups of type Y of about 1.

## Claims (Claims for the following Contracting State(s): ES)

1. Process for preparing perfluoropolyethers comprising sequences of statistically distributed perfluorooxyalkylene units selected from 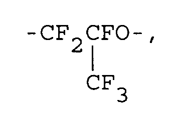 -CF₂CF₂O-, -CF₂O- and 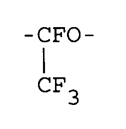 and having an acid group or derivative thereof, the other end group being a perhaloalkyl group containing one or two halogen atoms different from fluorine, said perfluoropolyethers being represented by the following general formulae: 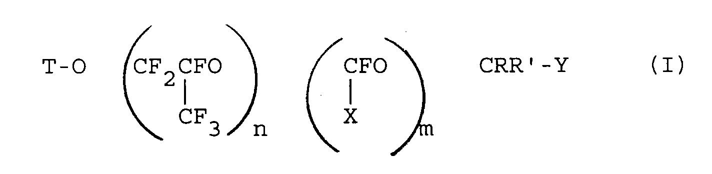 wherein: X is F or CF₃; R and R', the same or different from each other, are selected from F, Cl, Br and I;
Y is 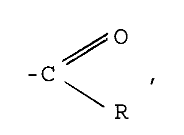 R being the same as defined above, or Y is the corresponding carboxyl group -COOH or a salt or ester group derived therefrom;
T is a perhaloalkyl group containing one or two atoms selected from Cl, Br and I and preferably represents ACF₂-, 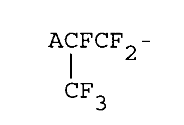 and ACF₂CF(CF₃)-,
wherein A is Cl, Br or I;
n ranges from 1 to 15;
and m/n is in the range of from 0.01 to 0.5;
T'-O (CF₂CF₂O)ₚ (CF₂O)ₛ CRR' Y (II)
wherein: T' = ACF₂CF₂-, ACF₂-, ACF₂CF(A)-, R, R', A and Y are the same as defined above for formula (I); p ranges from 1 to 20 and s/p is from 0.5 to 2; 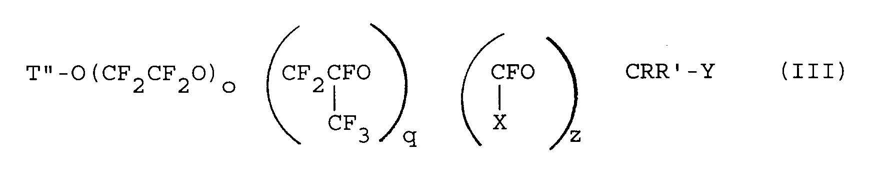 wherein: Y, X, R and R' are the same as defined above for formula (I);
T'' is equal to T or T', o and q are numbers, zero included, such that $\text{o+q = 1 to 20}$ , $\text{z/o+q}$ ranges from 0.01 to 0.05; and
n, m, p, s, o, q and z are average numbers;
said process comprising the photooxidation of C₃F₆ and/or C₂F₄ with gaseous oxygen, in the presence of a perhalogenated ethylene containing at least one halogen atom different from fluorine, at a temperature from -20° to -100°C, by irradiation of the liquid reaction mixture with ultraviolet light and the subsequent elimination of the peroxide groups by heat-treatment at 180° to 220°C, or the subsequent photochemical treatment with ultraviolet radiation, and optionally also comprising the hydrolysis, salification or alcoholysis of the acylhalide group formed.

2. Process according to claim 1 wherein perfluoropolyether mixtures are prepared which comprise at least 50% by weight of perfluoropolyethers of formulae I, II or III of claim 1, besides perfluoropolyethers corresponding to the above formulae, except that both of the end groups are either equal to T or T' or are equal to Y, said mixtures being characterized by a ratio of the number of end groups of types T, T' and T'' to the number of end groups of type Y of about 1.

## Patentansprüche (Patentansprüche für folgende(n) Vertragsstaat(en): BE, CH, DE, FR, GB, LI, NL, SE)

1. Perfluorpolyether, die Abfolgen von statistisch verteilten Perfluoroxyalkylen-Einheiten, die aus 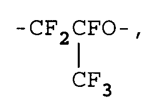 -CF₂CF₂O-, -CF₂O- und 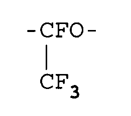 ausgewählt sind, umfassen und eine saure Endgruppe oder ein Derivat davon aufweisen, wobei die andere Endgruppe eine ein oder zwei von Fluor verschiedene Halogenatome enthaltende Perhalogenalkylgruppe ist, wobei diese Perfluorpolyether durch die folgenden allgemeinen Formeln dargestellt werden: 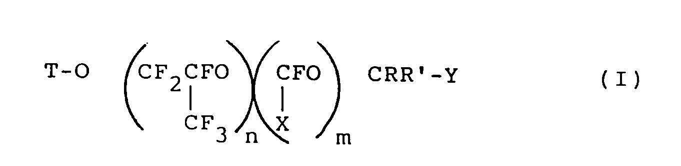 worin: X für F oder CF₃ steht; R und R', gleich oder verschieden voneinander, ausgewählt sind aus F, Cl, Br und I;
Y für 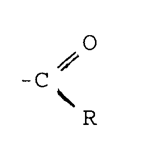 steht, wobei R gleich wie oben definiert ist, oder Y die entsprechende Carboxylgruppe -COOH oder eine davon abgeleitete Salz- oder Estergruppe bedeutet; T eine ein oder zwei aus Cl, Br und I ausgewählte Atome enthaltende Perhalogenalkylgruppe ist und vorzugsweise ACF₂-, 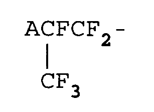 und ACF₂CF(CF₃)- darstellt, wobei A für Cl, Br oder I steht;
n im Bereich von 1 bis 15 liegt;
und m/n im Bereich von 0,01 bis 0,5 liegt;
T'-O (CF₂CF₂O)ₚ (CF₂O)ₛ CRR'Y (II)
worin: T' = ACF₂CF₂-, ACF₂-, ACF₂CF(A)-, R, R', A und Y dieselbe Bedeutung wie oben für Formel (I) angegeben aufweisen; p im Bereich von 1 bis 20 liegt und s/p 0,5 bis 2 beträgt; 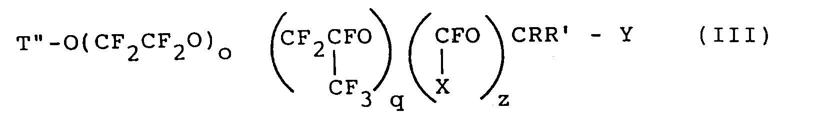 worin: Y, X, R und R' dieselbe Bedeutung wie oben für Formel (I) angegeben aufweisen;
T'' gleich T oder T' ist, o und q solche Zahlen, einschließlich 0, sind, daß $\text{o + q = 1 bis 20}$ , $\text{z/o + q}$ im Bereich von 0,01 bis 0,05 liegt, n, m, p, s, o, q und z durchschnittliche Zahlen sind.

2. Verfahren zur Herstellung der Perfluorpolyether nach Anspruch 1, umfassend die Photooxidation von C₃F₆ und/oder C₂F₄ mit gasförmigem Sauerstoff in Anwesenheit eines perhalogenierten Ethylens, das mindestens ein von Fluor verschiedenes Halogenatom enthält, bei einer Temperatur von -20 bis -100°C, durch Bestrahlung der flüssigen Reaktionsmischung mit ultraviolettem Licht und die anschließende Eliminierung der Peroxidgruppen durch Wärmebehandlung bei 180 bis 220°C oder die anschließende photochemische Behandlung mit UV-Strahlung, und gegebenenfalls auch die Hydrolyse, Überführung in Salz oder Alkoholyse der gebildeten Acylhalogenidgruppe umfassend.

3. Perfluorpolyether-Mischungen, umfassend mindestens 50 Gewichts-% Perfluorpolyether der Formeln I, II oder III von Anspruch 1, neben Perfluorpolyethern, die den obigen Formeln entsprechen, mit der Ausnahme, daß beide Endgruppen entweder gleich T oder T' oder gleich Y sind, wobei diese Mischungen durch ein Verhältnis der Anzahl der Endgruppen vom Typ T, T' und T'' zur Anzahl der Endgruppen vom Typ Y von etwa 1 gekennzeichnet sind.

## Patentansprüche (Patentansprüche für folgende(n) Vertragsstaat(en): ES)

1. Verfahren zur Herstellung von Perfluorpolyethern, die Abfolgen von statistisch verteilten Perfluoroxyalkylen-Einheiten, die aus 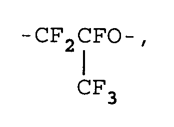 -CF₂CF₂O-, -CF₂O- und 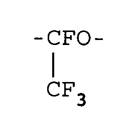 ausgewählt sind, umfassen und eine saure Gruppe oder ein Derivat davon aufweisen, wobei die andere Endgruppe eine ein oder zwei von Fluor verschiedene Atome enthaltende Perhalogenalkylgruppe ist, wobei diese Perfluorpolyether durch die folgenden allgemeinen Formeln dargestellt werden: 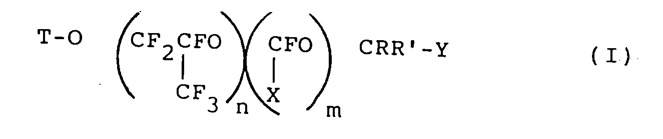 worin: X für F oder CF₃ steht; R und R', gleich oder verschieden voneinander, ausgewählt sind aus F, Cl, Br und I;
Y für 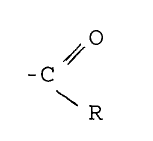 steht, wobei R gleich wie oben definiert ist, oder Y die entsprechende Carboxylgruppe -COOH oder eine davon abgeleitete Salz- oder Estergruppe bedeutet;
T eine ein oder zwei aus Cl, Br und I ausgewählte Atome enthaltende Perhalogenalkylgruppe ist und vorzugsweise ACF₂-, 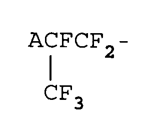 und ACF₂CF(CF₃)- darstellt, wobei A für Cl, Br oder I steht;
n im Bereich von 1 bis 15 liegt;
und m/n im Bereich von 0,01 bis 0,5 liegt;
T'-O (CF₂CF₂O)ₚ (CF₂O)ₛ CRR'Y (II)
worin: T' = ACF₂CF₂-, ACF₂-, ACF₂CF(A)-, R, R', A und Y dieselbe Bedeutung wie oben für Formel (I) angegeben aufweisen; p im Bereich von 1 bis 20 liegt und s/p 0,5 bis 2 beträgt; 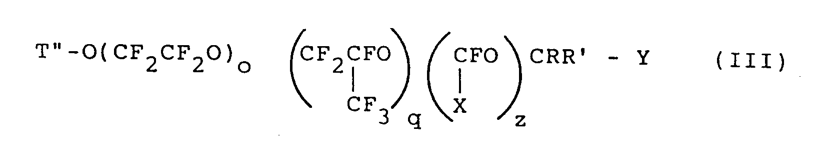 worin: Y, X, R und R' dieselbe Bedeutung wie oben für Formel (I) angegeben aufweisen;
T'' gleich T oder T' ist, o und q solche Zahlen, einschließlich 0, sind, daß $\text{o + q = 1 bis 20}$ , $\text{z/o + q}$ im Bereich von 0,01 bis 0,05 liegt; und n, m, p, s, o, q und z durchschnittliche Zahlen sind;
wobei das Verfahren umfaßt die Photooxidation von C₃F₆ und/oder C₂F₄ mit gasförmigen Sauerstoff in Anwesenheit eines perhalogenierten Ethylens, das mindestens ein von Fluor verschiedenes Halogenatom enthält, bei einer Temperatur von -20 bis -100°C, durch Bestrahlung der flüssigen Reaktionsmischung mit ultraviolettem Licht und die anschließende Eliminierung der Peroxidgruppen durch Wärmebehandlung bei 180 bis 220°C oder die anschließende photochemische Behandlung mit UV-Strahlung, und gegebenenfalls auch die Hydrolyse, Überführung in Salz oder Alkoholyse der gebildeten Acylhalogenidgruppe umfaßt.

2. Verfahren nach Anspruch 1, in welchem Perfluorpolyether-Mischungen hergestellt werden, welche mindestens 50 Gewichts-% Perfluorpolyether der Formeln I, II oder III nach Anspruch 1 neben Perfluorpolyethern umfassen, die den obigen Formeln entsprechen, mit der Ausnahme, daß beide Endgruppen entweder gleich T oder T' oder gleich Y sind, wobei diese Mischungen durch ein Verhältnis der Anzahl der Endgruppen der Typen T, T' und T'' zur Anzahl der Endgruppen vom Typ Y von etwa 1 gekennzeichnet sind.

## Revendications (Revendications pour l'(les) Etat(s) contractant(s) suivant(s): BE, CH, DE, FR, GB, LI, NL, SE)

1. Perfluoropolyéthers comprenant des séquences d'unités perfluorooxyalkytène distribuées statistiquement, choisies parmi 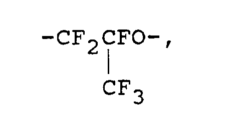 -CF₂CF₂O-, -CF₂O- et 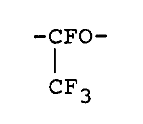 et ayant un groupe terminal acide ou un dérivé de celui-ci, l'autre groupe terminal étant un groupe perhaloalkyle contenant un ou deux atomes d'halogène différents du fluor, lesdits perfluoropolyéthers étant représentés par les formules générales suivantes : 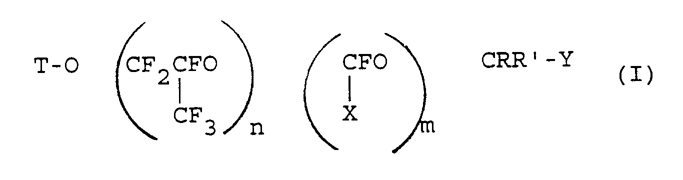 dans laquelle :
- X représente F ou CF₃ ;
- R et R', identiques ou différents l'un de l'autre, sont choisis parmi F, Cl, Br et I ;
- Y représente 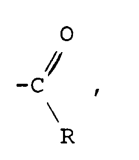 R étant le même que celui défini ci-dessus, ou Y représente le groupe carboxyle correspondant -COOH ou un groupe sel ou ester dérivé de celui-ci ;
- T représente un groupe perhaloalkyle contenant un ou deux atomes choisis parmi Cl, Br et I et de préférence représente ACF₂-, 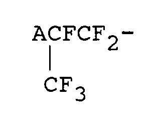 et ACF₂CF(CF₃)-, où A représente Cl, Br ou I ;
- n se situe dans une plage allant de 1 à 15 ; et
- m/n se situe dans la plage allant de 0,01 à 0,5 ;
T'-O (CF₂CF₂O)ₚ (CF₂O)ₛ CRR' Y (II)
dans laquelle :
- T' = ACF₂CF₂-, ACF₂-, ACF₂CF(A)- ;
- R, R', A et Y sont les mêmes que ceux définis ci-dessus pour la formule (I) ;
- p se situe dans une plage allant de 1 à 20 ; et
- s/p est de 0,5 à 2 ; 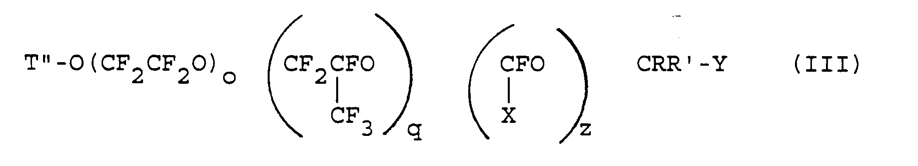
dans laquelle :
- Y, X, R et R' sont les mêmes que ceux définis ci-dessus pour la formule (I) ;
- T'' est égal à T ou T' ;
- o et q sont des nombres, zéro compris, tels que $\text{o + q = 1 à 20}$ , $\text{z / o + q}$ se situe dans une plage allant de 0,01 à 0,05 ;
n, m, p, s, o, q et z étant des nombres moyens.

2. Procédé de préparation des perfluoropolyéthers de la revendication 1, comprenant la photo-oxydation de C₃F₆ et/ou C₂F₄ avec de l'oxygène gazeux, en présence d'un éthylène perhalogéné contenant au moins un atome d'halogène différent du fluor, à une température de -20° à -100°C, par irradiation du mélange réactionnel liquide par une lumière ultraviolette, et l'élimination subséquente des groupes peroxyde par un traitement par la chaleur à 180° à 220°C, ou le traitement photochimique subséquent par un rayonnement ultraviolet, et facultativement comprenant également l'hydrolyse, la salification ou l'alcoolyse du groupe halogénure d'acyle formé.

3. Mélanges de perfluoropolyéthers, comprenant au moins 50% en poids de perfluoropolyéthers des formules (I), (II) ou (III) de la revendication 1, en dehors des perfluoropolyéthers correspondant aux formules ci-dessus excepté que les deux groupes terminaux sont ou bien égaux à T ou T', ou bien égaux à Y, lesdits mélanges étant caractérisés par un rapport du nombre de groupes terminaux de types T, T' et T'' au nombre de groupes terminaux de type Y d'environ 1.

## Revendications (Revendications pour l'(les) Etat(s) contractant(s) suivant(s): ES)

1. Procédé de préparation de perfluoropolyéthers comprenant des séquences d'unités perfluorooxyalkylène distribuées statistiquement, choisies parmi 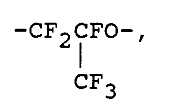 -CF₂CF₂O-, -CF₂O- et 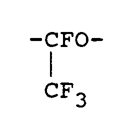 et ayant un groupe terminal acide ou un dérivé de celui-ci, l'autre groupe terminal étant un groupe perhaloalkyle contenant un ou deux atomes d'halogène différents du fluor, lesdits perfluoropolyéthers étant représentés par les formules générales suivantes : 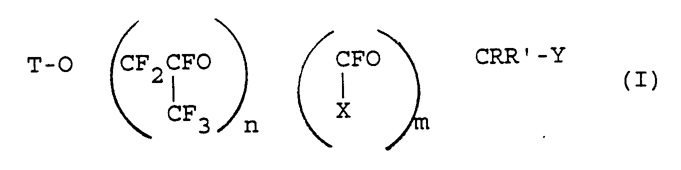 dans laquelle :
- X représente F ou CF₃ ;
- R et R', identiques ou différents l'un de l'autre, sont choisis parmi F, Cl, Br et I ;
- Y représente 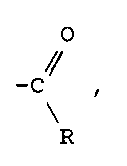 R étant le même que celui défini ci-dessus, ou Y représente le groupe carboxyle correspondant -COOH ou un groupe sel ou ester dérivé de celui-ci ;
- T représente un groupe perhaloalkyle contenant un ou deux atomes choisis parmi Cl, Br et I et de préférence représente ACF₂-, 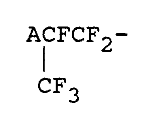 et ACF₂CF(CF₃)-, où A représente Cl, Br ou I :
- n se situe dans une plage allant de 1 à 15 ; et
- m/n se situe dans la plage allant de 0,01 à 0,5 ;
T'-O (CF₂CF₂O)ₚ (CF₂O)ₛ CRR' Y (II)
dans laquelle :
- T' = ACF₂CF₂-, ACF₂-, ACF₂CF(A)- ;
- R, R', A et Y sont les mêmes que ceux définis ci-dessus pour la formule (I) ;
- p se situe dans une plage allant de 1 à 20 ; et
- s/p est de 0,5 à 2 ; 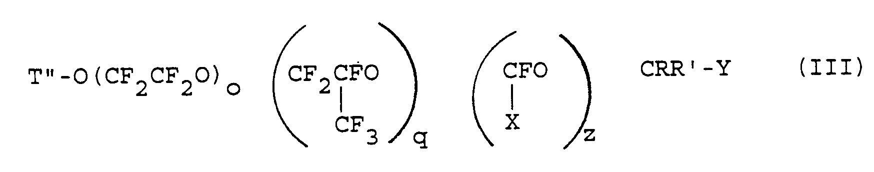
dans laquelle :
- Y, X, R et R' sont les mêmes que ceux définis ci-dessus pour la formule (I) ;
- T'' est égal à T ou T' ;
- o et q sont des nombres, zéro compris, tels que $\text{o + q = 1 à 20}$ , $\text{z / o + q}$ se situe dans une plage allant de 0,01 à 0,05 ;
n, m, p, s, o, q et z étant des nombres moyens ;
ledit procédé comprenant la photo-oxydation de C₃F₆ et/ou C₂F₄ avec de l'oxygène gazeux, en présence d'un éthylène perhalogéné contenant au moins un atome d'halogène différent du fluor, à une température de -20° à -100°C, par irradiation du mélange réactionnel liquide par une lumière ultraviolette, et l'élimination subséquente des groupes peroxyde par un traitement par la chaleur à 180° à 220°C, ou le traitement photochimique subséquent par un rayonnement ultraviolet, et facultativement comprenant également l'hydrolyse, la salification ou l'alcoolyse du groupe halogénure d'acyle formé.

2. Procédé selon la revendication 1, dans lequel on prépare des mélanges de perfluoropolyéthers qui comprennent au moins 50% en poids de perfluoropolyéthers des formules (I), (II) ou (III) de la revendication 1, en dehors des perfluoropolyéthers correspondant aux formules ci-dessus excepté que les deux groupes terminaux sont ou bien égaux à T ou T', ou bien égaux à Y, lesdits mélanges étant caractérisés par un rapport du nombre de groupes terminaux de types T, T' et T'' au nombre de groupes terminaux de type Y d'environ 1.
